# EUROPÄISCHE PATENTSCHRIFT

(11) **EP 0 397 048 B1**
(45) Veröffentlichungstag und Bekanntmachung des Hinweises auf die Patenterteilung: **31.08.1994**
(21) Anmeldenummer: 90108444.2
(22) Anmeldetag: 04.05.1990
(51) Int. Cl.: C07D 277/36

(54) **Verfahren zur Herstellung von 2-Mercapto-4-methyl-1,3-thiazol-5-yl-essigsäure und deren Ester**
Process for the preparation of 2-mercapto-4-methyl-1,3-thiazol-5-yl acetic acid and its esters
Procédé de préparation de l'acide 2-mercapto-4-méthyl-1,3-thiazol-5-yl acétique et de ses esters

(30) Priorität: 09.05.1989 DE 3915094
(43) Veröffentlichungstag der Anmeldung: 14.11.1990
(73) Patentinhaber: HOECHST AKTIENGESELLSCHAFT, 65926 Frankfurt am Main (DE)
(72) Erfinder: Korb, Gerhard, D-6452 Hainburg (DE); Flemming, Hans-Wolfram, Dr., D-6390 Usingen (DE)

(56) Entgegenhaltungen:
- EP-A- 0 064 256
- CHEMICAL ABSTRACTS, Band 50, Nr. 2, 25. January 1956, Seite 963,Zusammenfassung Nr. 963 a-d, Columbus, Ohio, US; Y. HARO et al.: "Synthesis of some thiazole derivatives from levulinic acid", & PHARM. BULL. (JAPAN), 2, 156-8 (1954)

## Beschreibung

Gegenstand der vorliegenden Erfindung ist ein Verfahren zur Herstellung von 2-Mercapto-4-methyl-1,3-thiazol-5-essigsäure und deren Alkylester der Formel I 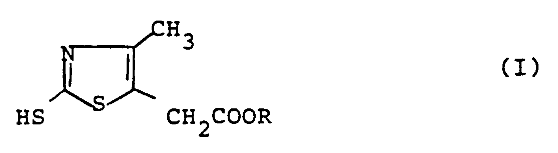
worin R Wasserstoff oder einen C₁-C₄-Alkylrest bedeutet, das dadurch gekennzeichnet ist, daß man einen Lävulinsäureester der Formel II 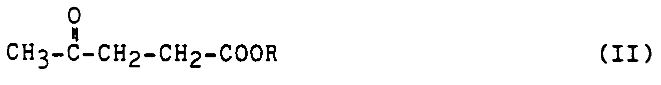
worin R einen C₁-C₄-Alkylrest bedeutet, mit 0,7 - 1,5 Mol eines Halogenierungsmittels pro Mol Lävulinsäureester bei einer Temperatur von -20°C bis +150°C umsetzt, aus dem erhaltenen Isomerengemisch das 3-Halogen-isomere der Formel III 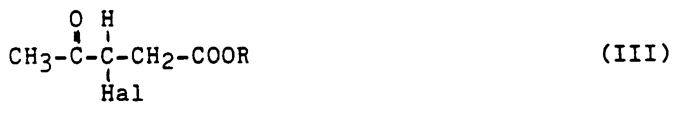
worin R die genannte Bedeutung hat und Hal ein Chlor-, Brom- oder Jodatom bedeutet,
abtrennt, dieses mit Ammoniumdithiocarbaminat der Formel IV 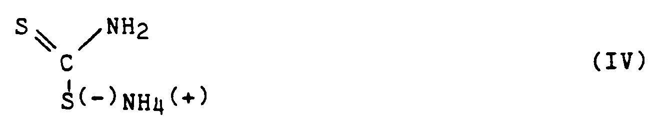
umsetzt zu der Verbindung der Formel I, worin R einen C₁-C₄-Alkylrest darstellt und gegebenenfalls den Ester der Formel I in die freie Säure überführt.

Die Verbindung der Formel I ist bereits bekannt. Sie kann z.B. als Molekülbaustein zur Herstellung des Antibiotikums Cefodizim (DE-OS 3 117 438) der Formel V 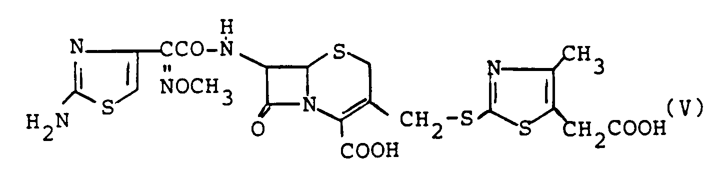
sowie auch zur Herstellung von Immunmodulatoren (DE-OS 3 508 665, DE-OS 3 508 666) verwendet werden.

Um ein spezifikationsgerechtes Cefodizim (V) zu erhalten, muß bei der Synthese eine Mercaptomethylthiazolsäure in genügend hoher Reinheit eingesetzt werden. Störend wirken insbesondere die isomere Verbindung 2-Mercaptothiazol-4-propionsäure (VI) 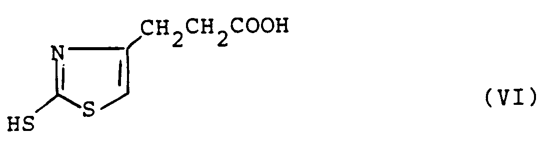
und färbende Verunreinigungen.

In der Literatur sind Verfahren zur Herstellung von Verbindungen der Formel (I) bekannt.
So wird z.B. gemäß Y. Hara und S. Fujise (Pharm. Bull. (Japan), Vol 2 (1954), S. 156-158) die Umsetzung von 3-Bromlävulinsäureethylester mit Ammoniumdithiocarbaminat zu einer Verbindung der vorliegenden Formel I, worin R den Ethylrest bedeutet, beschrieben. Dieses Verfahren jedoch führt zu sehr unbefriedigenden Ausbeuten. Auf der Stufe der Ester- und Säureherstellung werden größere Mengen Ether eingesetzt, der wegen der möglichen Peroxidbildung und leichten Entzündbarkeit sicherheitstechnisch sehr bedenklich ist. Zur Produktreinigung werden mehrfache Umkristallisationen aus Ethanol vorgenommen, was bei der Säure, wie nachgewiesen werden konnte, durch Autokatalyse zur erneuten Esterbildung führt.

Das Verfahren nach P. Lechat et al. (Therapie 26(3), 497-509 (1971)) ist mit den gleichen Nachteilen wie das vorher beschriebene Verfahren behaftet.

Bekannt ist aus der DE-OS 3 117 438 weiterhin ein Verfahren zur Herstellung von Verbindungen der oben angegebenen Formel I, das dadurch gekennzeichnet ist, daß 3-Halogenlävulinsäure zunächst verestert wird und der erhaltene Ester dann mit Ammoniumdithiocarbaminat umgesetzt wird.

Bei der vorausgehenden Halogenierung von Lävulinsäure, insbesondere der Chlorierung, z.B. mit Chlor oder Sulfurylchlorid, erhält man jedoch ein Gemisch aus 3-Chlorlävulinsäure, 3,3-Dichlorlävulinsäure, 5-Chlorlävulinsäure, 3,5-Dichlorlävulinsäure, 5,5-Dichlorlävulinsäure, weiteren nicht bekannten Nebenprodukten sowie nicht umgesetzter Lävulinsäure. Setzt man dieses Gemisch zur Herstellung der Mercaptomethylthiazolsäure der oben genannten Formel I mit Ammoniumdithiocarbaminat der oben genannten Formel IV um, so erhalt man ein gefärbtes, mit Nebenprodukten, insbesondere der 2-Mercapto-1,3-thiazol-4-propionsäure der Formel VI verunreinigtes Rohprodukt. Durch mehrere Reinigungsschritte (Umfällung und Umkristallisation aus organischen Lösemitteln) können die färbenden Verbindungen und das Isomere der Formel VI abgetrennt werden und man erhalt so eine verwendbare 2-Mercapto-4-methyl-thiazolessigsäure. Dabei werden jedoch sehr unbefriedigende Ausbeuten von nur etwa 15 - 20 % der Theorie, bezogen auf die eingesetzte Lävulinsäure, erzielt.
Weiterhin führt der Einsatz organischer Lösemittel zu hohen Aufarbeitungskosten und Umweltbelastungen.

Da die Dichlorverbindungen ebenfalls mit Ammoniumdithiocarbaminat reagieren, müssen in diesem Fall pro Mol eingesetzte 3-Chlor-Lävulinsäure ca. 2,5 Mol Ammoniumdithiocarbaminat eingesetzt werden.

Überraschenderweise wurde nun gefunden, daß man 2-Mercapto-4-methyl-1,3-thiazol-5-yl-essigsäure oder deren C₁-C₄-Alkylester in isomerenfreier Form und mit hohen Ausbeuten dadurch herstellen kann, daß man anstelle der Lävulinsäure den Lävulinsäurealkylester der Formel II 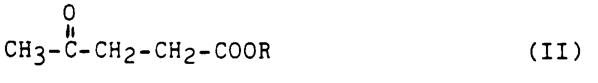
worin R C₁-C₄-Alkyl bedeutet, halogeniert und nach Abtrennung des resultierenden 3-Halogen-lävulinsäureesters durch Rektifikation diesen mit Ammoniumdithiocarbaminat umsetzt.

Die Einführung des Halogenatoms, das Chlor, Brom oder Jod sein kann, in 3-Stellung, erfolgt nach üblichen Methoden. Bevorzugt wird der Lävulinsäureester chloriert, und zwar insbesondere durch Umsetzung mit elementarem Chlor oder mit Sulfurylchlorid.

Die Reaktion kann ohne oder in Gegenwart eines anorganischen oder organischen Lösungsmittels erfolgen.

Die Menge an Halogenierungsmittel liegt dabei bei etwa 0,7 - 1,5 Mol pro Mol Lävulinsäureester, vorzugsweise jedoch bei etwa 1,0 - 1,3 Mol. Die Reaktionstemperatur kann in ziemlich breiten Bereichen schwanken. Im allgemeinen liegt sie zwischen -20°C und etwa +150°C, vorzugsweise jedoch zwischen -5°C und etwa +80°C.

Als bevorzugte anorganische Verdünnungsmittel sind Wasser oder Salzsäure beispielhaft genannt. Als bevorzugte organische Verdünnungsmittel sind hier beispielhaft angeführt:
C₅-C₇-aliphatische und C₆-C₈-cycloaliphatische Kohlenwasserstoffe wie z.B. Pentan, 2-Methylbutan, Hexan, 2,2-Dimethylbutan, 2,3-Dimethylbutan, 2-Methylpentan, 3-Methylpentan, Heptan, Cyclohexan, Methylcyclohexan, 1,2-Dimethylcyclohexan, 1,3-Dimethylcyclohexan, 1,4-Dimethylcyclohexan; aromatische Kohlenwasserstoffe wie z.B. Toluol, Xylole, Isopropylbenzol; aliphatische und aromatische Halogenkohlenwasserstoffe wie z.B. Tetrachlorethylen, 1,1,2,2- und 1,1,1,2-Tetrachlorethan, Methylenchlorid, Dichlorpropan, Tetrachlorkohlenstoff, 1,1,2-Trichlor-1,2,2-trifluorethan, 1,2-Dichlorethan, 1,1-Dichlorethan, Chlorbenzol.

Besonders bevorzugte Verdünnungsmittel sind aliphatische und cycloaliphatische Kohlenwasserstoffe.

Die Menge des eingesetzten Verdünnungsmittels, falls man nicht ohne Verdünnungsmittel arbeitet, kann in weiten Bereichen schwanken.
Bei der aufgezeigten Chlorierungsmethode entsteht bevorzugt der gewünschte 3-Chlorlävulinsäureester (VII). Als Nebenprodukt entstehen - je nach Variation der Bedingungen - 3,3-Dichlorlävulinsäureester (VIII), 5-Chlorlävulinsäureester (IX), 3,5-Dichlorlävulinsäureester (X) und 5,5-Dichlorlävulinsäureester (XI) in nennenswerten Mengen. Außerdem kann nicht umgesetzter Lävulinsäureester im Chlorierungsgemisch noch vorhanden sein. 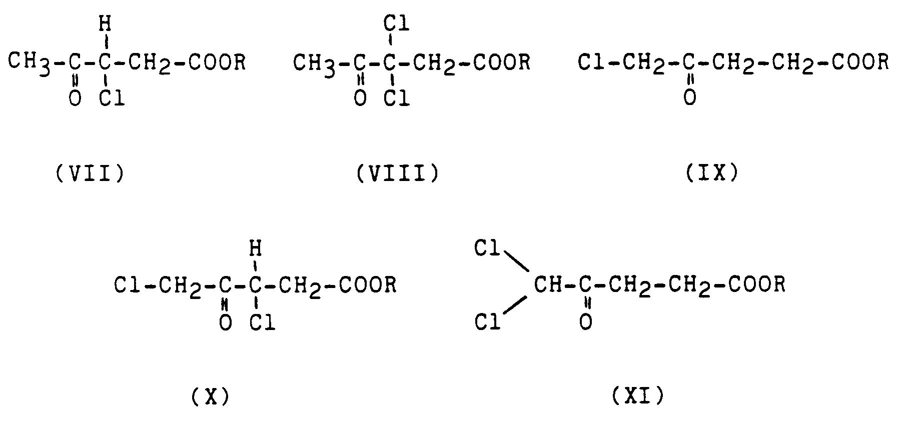
Aus dem Reaktionsgemisch trennt man den entstandenen Chlorwasserstoff durch Wasserwäsche mit anschließender Phasentrennung, Ausblasen mit Trägergas oder Destillation ab.

Verwendetes Verdünnungsmittel kann ebenfalls durch Phasentrennung oder Destillation nach in Laboratorien üblichen Verfahren abgetrennt werden.

Das eigentliche, so vorgereinigte Reaktionsgemisch, unterwirft man einer Rektifikation unter Vakuum. Im Gegensatz zum Reaktionsgemisch, das bei der Halogenierung z.B. der Chlorierung der Lävulinsäure, entsteht, gelingt es bei dem erfindungsgemäßen Verfahren, alle störenden, zu Nebenprodukten führenden Verunreinigungen, abzutrennen. Dabei trennt man das Gemisch wie folgt:
- Vorlauf:: Lävulinsäureester als Hauptbestandteil
- Hauptlauf:: 3-Chlorlävulinsäureester als Hauptbestandteil, verunreinigt mit 3,3-Dichlorlävulinsäureester
- Rückstand:: 5-Chlorlävulinsäureester, 3,5-Dichlorlävulinsäureester, 5,5-Dichlorlävulinsäureester und sonstige chlorierte Nebenprodukte
Dabei kann der Vorlauf bei einer erneuten Chlorierung zur Ausbeutesteigerung wieder eingesetzt werden.

Um hohe Ausbeuten an 3-Chlorlävulinsäureester (VII) zu erhalten, muß bei der Destillation der überwiegende Teil des 3,3-Dichlorlävulinesters (VIII) mit abdestilliert werden, da diese Verbindung keine große Siedepunktsdifferenz zu (VII) hat.

Überraschenderweise reagiert (VIII) in der Folgeumsetzung unter den gewählten Bedingungen nicht mit Ammoniumdithiocarbaminat zu störenden Thiazolderivaten. Erfindungsgemäß setzt man den Hauptlauf aus der Rektifikation mit Ammoniumdithiocarbaminat in Wasser, zweckmäßigerweise unter Zusatz eines mit Wasser nur schwer bzw. nicht mischbaren Lösemittels, gegebenenfalls unter Zusatz eines Phasentransferkatalysators, um. Nach Ansäuern mit einer Mineralsäure isoliert man den Ester nach üblichen Laborverfahren.

Die Reaktionstemperatur liegt im allgemeinen im Bereich von etwa -10 bis +60°C, vorzugsweise jedoch zwischen +10 und +30°C. Als mit Wasser nur schwer bzw. nicht mischbare Lösemittel werden beispielhaft genannt:
Aliphatische und cycloaliphatische Kohlenwasserstoffe wie z.B. Pentan, 2-Methylbutan, 2,2-Dimethylbutan, 2,3-Dimethylbutan, 2-Methylpentan, 3-Methylpentan, Heptan, Cyclohexan, Methylcyclohexan, 1,2-Dimethylcyclohexan, 1,3-Dimethylcyclohexan, 1,4-Dimethylcyclohexan; aromatische Kohlenwasserstoff wie z.B. Toluol, Xylol, Isopropylbenzol; aliphatische und aromatische Halogenkohlenwasserstoffe wie z.B. Tetrachlorethylen, 1,1,2,2- und 1,1,1,2-Tetrachlorethan, Methylenchlorid, Dichlorpropan, Tetrachlorkohlenstoff, 1,1,2-Trichlor-1,2,2-trifluorethan, Trichlorfluormethan, 1,2-Dichlorethan, 1,1-Dichlorethan, Chlorbenzol; Alkohole wie z.B. Butanol, Pentanol, Isobutanol usw.; Ester wie z.B. Butylacetat, Amylacetat usw.; Ether wie z.B. Diisopropylether, Diisoamylether, Methyl-t-butylether, Di-n-butylether, Ethylenglykoldimethylether, Tetrahydrofuran, Dioxan, Anisol.

Je nach Löslichkeit des herzustellenden Mercaptomethylthiazolessigsäureesters werden Art und Menge des mit Wasser nicht oder nur schwer löslichen Lösmittels gewählt.

Zur Reaktionsbeschleunigung kann der Einsatz von Phasentransferkatalysatoren vorteilhaft sein. Beispielhaft genannt seien Ammoniumsalze, wie Tetrabutylammoniumchlorid, Tetrabutylammoniumbromid, Tetrabutylammoniumjodid, Tetrabutylammoniumhydrogensulfat, Benzyl-trimethylammoniumbromid, Benzyl-dimethyl-n-dodecylammoniumbromid, Trioctyl-methyl-ammoniumchlorid oder quartäre Phosphoniumverbindungen, wie beispielsweise Ethyl-trioctyl-phosphoniumbromid und Hexadecyl-tributylphosphoniumbromid.

Als Mineralsäuren sind beispielhaft genannt: Salzsäure, Bromwasserstoffsäure, Schwefelsäure, Salpetersäure usw. Der so erhaltene Mercaptomethylthiazolester ist praktisch isomerenfrei und kann durch Verseifung in wäßriger Lösung und anschließendes Ansäuern in die freie Mercaptomethylthiazolessigsäure überführt werden. Die Verseifungstemperatur kann in weiten Bereichen schwanken. Im allgemeinen liegt sie bei etwa -10 bis etwa +100°C, vorzugsweise bei 15 - 40°C.

Zur Verseifung verwendet man wäßrige Lösungen von Alkalihydroxiden, wie z.B. Natriumhydroxid und Kaliumhydroxid, vorzugsweise etwa 2 Mol auf 1 Mol Ester.

Als Mineralsäuren sind beispielhaft genannt: Salzsäure, Bromwasserstoffsäure, Schwefelsäure, Salpetersäure. Die so hergestellte Mercaptomethylthiazolsäure genügt voll den Anforderungen für die Weiterverarbeitung zu Cefodizim (V).

Die folgenden Beispiele sollen die Erfindung erläutern, ohne sie jedoch einzuschränken.

### Beispiel 1

### Herstellung von 2-Mercapto-4-methyl-1,3-thiazol-5-yl-essigsäuremethylester

130 g Lävulinsäuremethylester werden mit 390 ml n-Heptan versetzt. Bei ca. 70°C tropft man innerhalb von 15 Minuten 92 ml Sulfurylchlorid zu und rührt noch 3 Stunden nach. Dann destilliert man das n-Heptan unter Normaldruck ab und erhält als Rückstand ca. 168 g Chlorierungsgemisch. Anschließend trennt man das Chlorierungsgemisch unter Vakuum bei ca. 50 mbar über eine Raschigkolonne.

| | |
|---|---|
| Vorlauf: | 13 g (Gehalt 10 g Lävulinsäuremethylester) |
| Hauptlauf: | 120 g |
| Rückstand: | 65 g |

Den Hauptlauf versetzt man unter guter Rührung mit 50 ml n-Butanol, 100 ml Wasser und 68 g Ammoniumdithiocarbaminat, gibt 1 g Tetrabutylammoniumchlorid zu und läßt 3 Stunden bei 25-30°C nachreagieren. Durch Zugabe von ca. 10 ml Salzsäure, 37 %, stellt man einen pH-Wert von etwa 0,5 - 1,0 ein, kühlt auf 0 - 5°C ab und isoliert den entstandenen Ester. Der Ester wird mit kaltem n-Butanol und Wasser gewaschen.

Nach Trocknung erhält man 111,6 g 2-Mercapto-4-methyl-1,3-thiazol-5-yl-essigsäuremethylester. Das entspricht einer Ausbeute von 59,6 % der Theorie, bezogen auf den verbrauchten Lävulinsäuremethylester.

### Beispiel 2

### Herstellung von 2-Mercapto-4-methyl-1,3-thiazol-5-yl-essigsäureethylester

144 g Lävulinsäureethylester werden auf 0 - 5°C abgekühlt. Bei dieser Temperatur werden innerhalb von 3 Stunden 74 g Chlor eingeleitet. Anschließend wird das Chlorierungsgemisch auf 200 ml Eiswasser gegeben. Durch Abtrennen der organischen Phase erhält man 180 g Chlorierungsgemisch, das anschließend unter Vakuum bei ca. 15 mbar über eine Kolonne, gefüllt mit Raschigringen, destillativ gereinigt wird.

| | |
|---|---|
| Vorlauf: | 22 g (Gehalt 18 g Lävulinsäureethylester) |
| Hauptlauf: | 105 g |
| Rückstand: | 53 g |

Den Hauptlauf versetzt man unter guter Rührung mit 45 ml Butanol, 90 ml Wasser und 55 g Ammoniumdithiocarbaminat, fügt 1 g Tetrabutylammoniumchlorid hinzu und läßt noch 3 Stunden bei 25 - 30°C nachreagieren. Durch Zugabe von ca. 8 ml Salzsäure, 37 %, wird der pH-Wert auf ca. 0,5 bis 1,0 eingestellt, auf 0 - 5°C abgekühlt und anschließend der entstandene Ester abgesaugt. Nach Waschen mit kaltem n-Butanol und Wasser wird getrocknet. Man erhält 98,6 g 2-Mercapto-4-methylthiazol-5-yl-essigsäureethylester. Das entspricht einer Ausbeute von 51,9 % der Theorie, bezogen auf den verbrauchten Lävulinsäureethylester.

### Beispiel 3

### Herstellung von 2-Mercapto-4-methyl-1,3-thiazol-5-yl-essigsäure

108,5 g 2-Mercapto-4-methyl-1,3-thiazol-5-yl-essigsäureethylester werden in 200 ml Wasser suspendiert und bei 20 - 30°C mit 127 g Natronlauge, 33 %, versetzt. Anschließend wird noch 2 - 3 Stunden bei dieser Temperatur nachgerührt. Dann wird durch Zugabe von 54 g Salzsäure, 37 %, der pH-Wert auf 7 - 7,5 eingestellt. Nach Klärung mit 5 g Aktivkohle säuert man durch weitere Zugabe von 51 g Salzsäure, 37 %, auf pH 0,5 - 1,0 an. Die ausgefallene 2-Mercapto-4-methyl-1,3-thiazol-5-yl-essigsäure wird nach Abkühlen auf 0 - 5°C abgesaugt und dann mit kaltem Wasser gewaschen. Nach Trocknung erhält man 90,7 g reine Säure. Das entspricht einer Ausbeute von 96 % der Theorie, bezogen auf den eingesetzten 2-Mercapto-4-methyl-1,3-thiazol-5-yl-essigsäureethylester.

## Patentansprüche

1. Verfahren zur Herstellung von 2-Mercapto-4-methyl-1,3-thiazol-5-yl-essigsäure oder deren Alkylester der Formel I 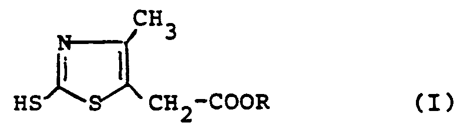 worin R Wasserstoff oder C₁-C₄-Alkyl bedeutet, dadurch gekennzeichnet, daß man
a) Lävulinsäureester der Formel II 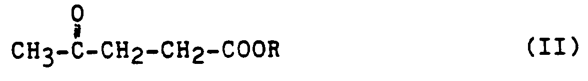 worin R C₁-C₄-Alkyl bedeutet, mit 0,7-1,5 Mol eines Halogenierungsmittels pro Mol Lävulinsäureester bei einer Temperatur von -20°C bis +150°C umsetzt,
b) aus dem erhaltenen Reaktionsgemisch durch Rektifikation eine Fraktion, die im wesentlichen aus der gewünschten 3-Halogenverbindung der Formel III 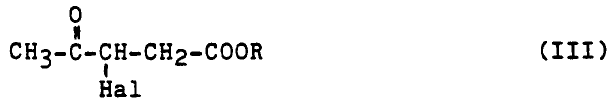 und zu einem geringen Teil aus der 3,3-Dihalogen-Verbindung der Formel XIII 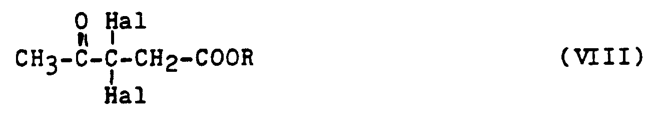 besteht,
worin R die oben genannte Bedeutung hat und Hal Chlor, Brom oder Jod bedeutet,
abtrennt,
c) die nach Schritt b) erhaltene Fraktion mit Ammoniumdithiocarbaminat umsetzt zu einer Verbindung der Formel I, worin R C₁-C₄-Alkyl bedeutet, und
d) gegebenenfalls den erhaltenen Ester nach Üblichen Methoden in eine Verbindung der Formel I Überführt, worin R Wasserstoff bedeutet.

2. Verfahren nach Anspruch 1, dadurch gekennzeichnet, daß die Verbindung der Formel II mit einem Chlorierungsmittel umgesetzt wird.

3. Verfahren nach Anspruch 2, dadurch gekennzeichnet, daß die Umsetzung mit elementarem Chlor oder mit Sulfurylchlorid mit oder ohne Verdünnungsmittel erfolgt.

4. Verfahren nach Anspruch 3, dadurch gekennzeichnet, daß mit Sulfurylchlorid in Gegenwart eines aliphatischen oder cycloaliphatischen Kohlenwasserstoffs chloriert wird.

5. Verfahren nach Anspruch 1, dadurch gekennzeichnet, daß die Rektifikation bei vermindertem Druck durchgeführt wird.

6. Verfahren nach Anspruch 1, dadurch gekennzeichnet, daß die Umsetzung mit Ammoniumdithiocarbaminat unter Zusatz eines mit Wasser nicht oder schwer mischbaren Lösungsmittels durchgeführt wird.

7. Verfahren nach Anspruch 6, dadurch gekennzeichnet, daß als Lösungsmittel ein C₄- oder C₅-Alkanol verwendet wird.

8. Verfahren nach Anspruch 6, dadurch gekennzeichnet, daß die Umsetzung mit Ammoniumdithiocarbaminat in Gegenwart eines Phasentransferkatalysators durchgeführt wird.

9. Verfahren nach Anspruch 8, dadurch gekennzeichnet, daß als Phasentransferkatalysator ein quartäres Ammoniumsalz oder eine quartäre Phosphoniumverbindung verwendet wird.

10. Verfahren nach Ansprüchen 6 bis 9, dadurch gekennzeichnet, daß die Umsetzung bei einer Temperatur von 10 - 30°C erfolgt.

## Claims

1. A process for the preparation of 2-mercapto-4-methyl-1,3-thiazol-5-ylacetic acid or an alkyl ester thereof, of the formula I 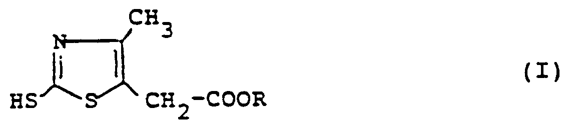 in which R denotes hydrogen or C₁-C₄-alkyl, which comprises
a) reacting a levulinic acid ester of the formula II 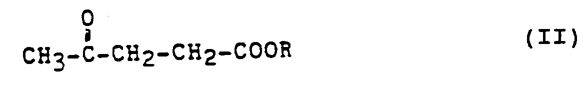 in which R denotes C₁-C₄-alkyl with 0.7 - 1.5 mol of a halogenating agent per mole of levulinic acid ester at a temperature of from -20°C to +150°C
b) separating off from the resulting reaction mixture, by rectification, a fraction which essentially comprises the desired 3-halo compound of the formula III 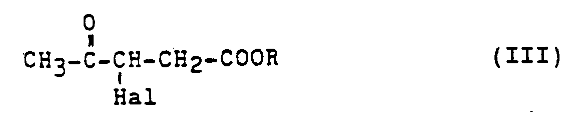 and in a small amount the 3,3-dihalo compound of the formula XIII 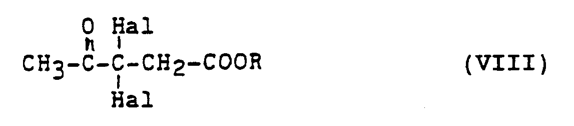 in which R has the abovementioned meaning and Hal denotes chlorine, bromine or iodine,
c) reacting the fraction obtained according to step b) with ammonium dithiocarbamate to give a compound of the formula I in which R denotes C₁-C₄-alkyl, and
d) if appropriate converting the resulting ester into a compound of the formula I in which R denotes hydrogen, by customary methods.

2. The process as claimed in claim 1, wherein the compound of the formula II is reacted with a chlorinating agent.

3. The process as claimed in claim 2, wherein the reaction is carried out with elemental chlorine or with sulfuryl chloride with or without a diluent.

4. The process as claimed in claim 3, wherein the chlorination is carried out with sulfuryl chloride in the presence of an aliphatic or cycloaliphatic hydrocarbon.

5. The process as claimed in claim 1, wherein the rectification is carried out under reduced pressure.

6. The process as claimed in claim 1, wherein the reaction with ammonium dithiocarbamate is carried out with addition of a solvent which is immiscible or sparingly miscible with water.

7. The process as claimed in claim 6, wherein a C₄- or C₅-alkanol is used as the solvent.

8. The process as claimed in claim 6, wherein the reaction with ammonium dithiocarbamate is carried out in the presence of a phase transfer catalyst.

9. The process as claimed in claim 8, wherein a quaternary ammonium salt or a quaternary phosphonium compound is used as the phase transfer catalyst.

10. The process as claimed in claims 6 to 9, wherein the reaction is carried out at a temperature of 10 - 30°C.

## Revendications

1. Procédé pour la préparation de l'acide 2-mercapto-4-méthyl-1,3-thiazol-5-yl-acétique ou de ses esters alkyliques de formule I 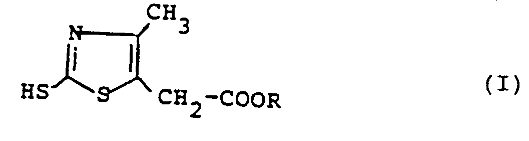 dans laquelle R représente un atome d'hydrogène ou un groupe alkyle en C₁-C₄, caractérisé en ce que
a) on fait réagir un ester d'acide lévulique de formule II 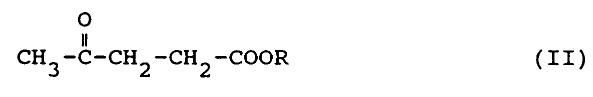 dans laquelle R représente un groupe alkyle en C₁-C₄-, avec 0,7-1,5 mole d'un agent d'halogénation par mole d'ester d'acide lévulique, à une température de -20 à +150°C,
b) à partir du mélange réactionnel obtenu, on sépare par rectification une fraction qui consiste essentiellement en le composé 3-halogéné recherché, de formule III 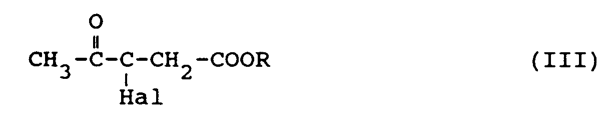 et, en une faible proportion, en le composé 3,3-di-halogéné de formule VIII 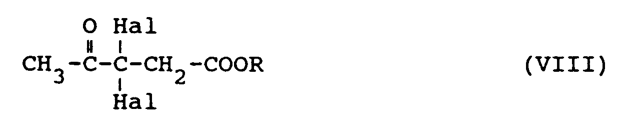 dans laquelle R a la signification donnée ci-dessus et Hal représente le chlore, le brome ou l'iode,
c) on fait réagir avec du dithiocarbamate d'ammonium la fraction obtenue après l'étape b), pour aboutir à un composé de formule I dans lequel R représente un groupe alkyle en C₁-C₄, et
d) éventuellement on convertit l'ester obtenu, selon des méthodes usuelles, en un composé de formule I dans lequel R représente un atome d'hydrogène.

2. Procédé selon la revendication 1, caractérisé en ce que l'on fait réagir le composé de formule II avec un agent de chloration.

3. Procédé selon la revendication 2, caractérisé en ce que la réaction s'effectue avec du chlore élémentaire ou avec du chlorure de sulfuryle, avec ou sans diluant.

4. Procédé selon la revendication 3, caractérisé en ce que l'on effectue la chloration avec du chlorure de sulfuryle en présence d'un hydrocarbure aliphatique ou cycloaliphatique.

5. Procédé selon la revendication 1, caractérisé en ce que la rectification est effectuée sous pression réduite.

6. Procédé selon la revendication 1, caractérisé en ce que la réaction avec le dithiocarbamate d'ammonium est effectuée avec addition d'un solvant non miscible ou peu miscible à l'eau.

7. Procédé selon la revendication 6, caractérisé en ce que l'on utilise comme solvant un alcanol en C₄ ou C₅.

8. Procédé selon la revendication 6, caractérisé en ce que la réaction avec le dithiocarbamate d'ammonium est effectuée en présence d'un catalyseur de transfert de phase.

9. Procédé selon la revendication 8, caractérisé en ce que l'on utilise comme catalyseur de transfert de phase un sel d'ammonium quaternaire ou un dérivé de phosphonium quaternaire.

10. Procédé selon les revendications 6 à 9, caractérisé en ce que la réaction est effectuée à une température de 10 à 30°C.
